# EUROPEAN PATENT APPLICATION

(11) **EP 4 729 267 A1**
(43) Date of publication of application: **22.04.2026**
(21) Application number: 24863280.4
(22) Date of filing: 06.09.2024
(51) Int. Cl.: B29C 45/00, B29C 45/16, B29C 45/18, B29C 45/26, B29D 23/00, B29K 69/00, B29K 75/00, B29L 31/00

(54) **ENDOSCOPE**

(30) Priority: 08.09.2023 KR 20230119414
(71) Applicant: LG Electronics Inc., Yeongdeungpo-gu Seoul 07336 (KR)
(72) Inventor: KIM, Wonsu, Seoul 06772 (KR); HWANG, Changkyu, Seoul 06772 (KR); KIM, Soonbeom, Seoul 06772 (KR)
(74) Representative: Vossius & Partner Patentanwälte Rechtsanwälte mbB
(86) International application number: PCT/KR2024/013557
(87) International publication number: WO 2025/053701

(57) **Abstract**

There is disclosed an endoscope, including: an insertion unit including an illumination and imaging unit and an insertion tube; and an operating unit provided with an operating knob and configured to perform a bending operation of the insertion tube according to an operation of the operating knob, wherein the insertion tube includes a bending part configured to be bent according to the operation of the operating unit, and the bending part includes a bending section injection-molded of a first material and a connection portion injection-molded of a second material different from the first material and connecting a plurality of the bending sections, thereby having an effect of manufacturing the bending part capable of providing bending performance at a low cost.

## Description

### [Technical Field]

Embodiments of the present invention relate to an endoscope, and more particularly, to an endoscope in which an insertion unit and an operating unit are detachably provided so that a disposable insertion unit can be replaced and used.

### [Background Art]

A medical endoscope is a device used to check a condition of an affected area or perform a procedure by being directly inserted into internal body tissues of a subject for examination or surgery.

A typical endoscope includes an articulated assembly disposed at a distal end of a flexible insertion tube to allow an insertion direction to be adjusted during insertion into a human body. A distal end body equipped with an image sensor and a light source is provided at an end of the articulated assembly. A handle body may be provided at the other end of the insertion tube. The handle body is connected to an image processing device (not shown) through a separate cable and a connector.

Dials and buttons used for controlling the direction of the articulated assembly and performing procedures are disposed on the handle body.

A pair of dials are disposed to adjust a direction of travel of the distal end body relative to a longitudinal direction of the insertion tube in vertical and horizontal directions. A sprocket and a chain are provided inside the handle body to convert rotational movement of the dials into linear movement. The chain is connected to an end of the articulated assembly through a separate wire. As a result, rotation of the dials is converted into linear movement of the wire via the sprocket and the chain, and the articulated assembly is bent according to the linear movement of the wire. This allows an operator of the endoscope to adjust the direction of travel of the distal end body during insertion of the insertion tube into the human body.

Here, because the endoscope is inserted into the human body, thorough hygiene management is required, and cleaning/disinfection must be performed before and after examination or surgery. In some cases, it may be impossible or undesirable to reuse the portion actually inserted into the human body, that is, the insertion tube and the articulated assembly. In this case, since a conventional endoscope is formed of a single body entirely, a problem arises in that the entire endoscope apparatus must be replaced. Furthermore, even if reuse is possible, cleaning and disinfection can be performed more simply if only a part of the endoscope can be separated, rather than the entire endoscope.

Meanwhile, a bending part of a conventional endoscope requires flexible operation during bending driving while simultaneously ensuring rigidity. To this end, the conventional endoscope is provided with an articulated structure formed of metal, which is connected using welding or rivets to enable bending driving.

However, since the articulated structure formed of metal as described above incurs high manufacturing costs, there is a limitation in that it is not suitable for a disposable endoscope that is replaced after a single use.

### [Summary of Invention]

### [Technical Problem]

Accordingly, one object of the embodiments of the present invention is to solve the above-noted disadvantages of the prior art, and to provide an endoscope in which a bending part may be manufactured using a material that has both rigidity and flexibility and has a low cost.

Further, another object of the present invention is to provide an endoscope including a bending part that may be manufactured at a low cost through a simple process.

### [Technical Solution]

In order to achieve the above objects, an endoscope according to the present invention includes: an insertion unit having an illumination and imaging unit and an insertion tube; and an operating unit provided with an operating knob and configured to perform a bending operation of the insertion tube according to an operation of the operating knob, wherein the insertion tube includes a bending part configured to be bent according to the operation of the operating unit, and the bending part includes: a bending section injection-molded of a first material; and a connection portion injection-molded of a second material different from the first material and connecting a plurality of the bending sections.

In this case, the bending section may be formed in a ring shape.

In this case, the connection portions may be formed as a pair at both longitudinal ends of the bending section, respectively.

In addition, the connection portions may be disposed with a constant phase difference along a circumferential direction of the bending section.

With such a configuration, the plurality of bending sections may be rotated relative to each other about the connection portion as an axis.

Meanwhile, the first material may be a polycarbonate-based material.

Further, the second material may be a polyurethane-based material. For example, the second material may be thermoplastic polyurethane.

Meanwhile, the bending section and the connection portion may be integrally formed.

In this case, the bending section and the connection portion may be formed by being simultaneously injected.

Meanwhile, a wire configured to linearly reciprocate according to the operation of the operating knob may pass through an inside of the bending section.

In this case, the first material may have higher rigidity than the second material.

Further, the second material may have higher flexibility than the first material.

### [Advantageous Effects]

As described above, according to the endoscope of the present invention, there is an effect that a bending part may be manufactured at a low cost by providing the bending part consisting of bending sections made of a polycarbonate-based material having rigidity and connection portions made of a urethane-based material having flexibility.

Further, since the bending part is manufactured by injecting two types of materials at once using a single mold, there is an effect that the bending part may be manufactured at a low cost.

### [Description of Drawings]

FIG. 1 is a perspective view for explaining an endoscope according to an embodiment of the present invention;
FIG. 2 is a perspective view for explaining a detachable relationship between an operating unit and an insertion unit in the endoscope according to an embodiment of the present invention;
FIG. 3 is a top view of FIG. 2;
FIG. 4 is a view for explaining a coupling method of a first coupling part and a second coupling part in the endoscope according to an embodiment of the present invention;
FIG. 5 is an enlarged view of FIG. 4 as viewed from another angle;
FIG. 6 is a view for explaining a state in which the first coupling part and the second coupling part are coupled in the endoscope according to an embodiment of the present invention;
FIGS. 7 to 13 are views for explaining a structure for guiding an initial position of a wire frame in the endoscope according to an embodiment of the present invention;
FIG. 14 is a perspective view of FIG. 2 as viewed from another angle;
FIG. 15 is a perspective view for explaining an illumination and imaging unit in the endoscope according to an embodiment of the present invention;
FIG. 16 is a perspective view for explaining an internal structure of the illumination and imaging unit in the endoscope according to an embodiment of the present invention
FIG. 17 is a view for explaining a heat sink in an endoscope according to an embodiment of the present invention; and
FIGS. 18 and 19 are views for explaining a bending part in the endoscope according to an embodiment of the present invention.

### [Description of Embodiments]

Hereinafter, preferred embodiments of the present invention will be described in detail with reference to the accompanying drawings.

The present invention may be subject to various changes and may have various embodiments, and specific embodiments will be illustrated in the drawings and described in detail in the detailed description. This is not intended to limit the present invention to specific embodiments, and it should be interpreted to include all changes, equivalents, and substitutes included in the spirit and technical scope of the present invention.

In describing the present invention, terms such as "first" and "second" may be used to describe various components, but the components may not be limited by the terms. These terms are only used for the purpose of distinguishing one component from another. For example, a first component may be named a second component without departing from the scope of rights of the present invention, and similarly, a second component may also be named a first component.

The term "and/or" may include a combination of a plurality of related listed items or any of the plurality of related listed items.

When a component is referred to as being "connected" or "coupled" to another component, it may be directly connected or coupled to the other component, but it should be understood that other components may exist in between. On the other hand, when a component is referred to as being "directly connected" or "directly coupled" to another component, it should be understood that no other components exist in between.

The terms used in the present application are only used to describe specific embodiments and are not intended to limit the present invention. Singular expressions may include plural expressions unless the context clearly indicates otherwise.

In the present application, terms such as "comprise" or "have" are intended to designate that features, numbers, steps, operations, components, parts, or combinations thereof described in the specification exist, and it should be understood that they do not preclude the presence or addition of one or more other features, numbers, steps, operations, components, parts, or combinations thereof.

Unless otherwise defined, all terms used herein, including technical or scientific terms, may have the same meaning as commonly understood by those of ordinary skill in the art to which the present invention belongs. Terms such as those defined in commonly used dictionaries may be interpreted as having a meaning consistent with the meaning in the context of the related technology, and unless explicitly defined in the present application, they may not be interpreted in an ideal or excessively formal sense.

In addition, the following embodiments are provided to more completely explain to those of ordinary skill in the art, and the shapes and sizes of elements in the drawings may be exaggerated for a clearer explanation.

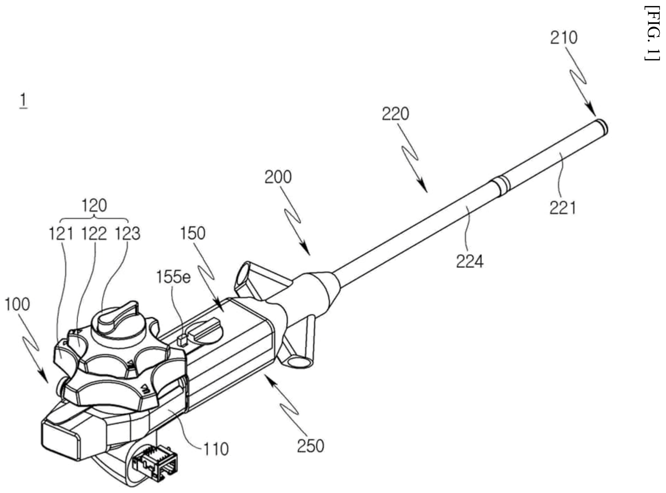

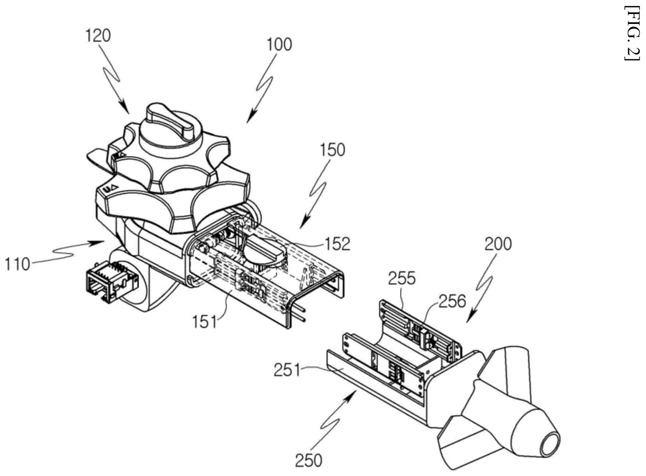

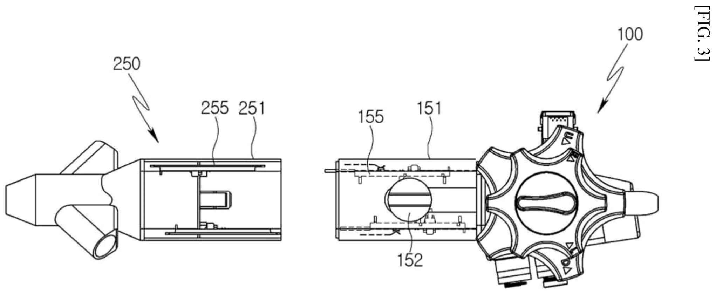

FIG. 1 is a perspective view for explaining an endoscope according to an embodiment of the present invention, FIG. 2 is a perspective view for explaining a detachable relationship between an operating unit and an insertion unit in the endoscope according to an embodiment of the present invention, and FIG. 3 is a top view of FIG. 2.

Referring to FIGS. 1 to 3, an endoscope 1 according to an embodiment of the present invention may be a disposable endoscope. Specifically, the endoscope 1 according to an embodiment of the present invention includes an operating unit 100 and an insertion unit 200. At this time, the operating unit 100 and the insertion unit 200 may be detachably coupled to each other by coupling parts 150 and 250. In this case, the coupling parts 150 and 250 include a first coupling part 150 provided in the operating unit 100 and a second coupling part 250 provided in the insertion unit 200.

The coupling parts 150 and 250 in the present invention are configured to convert a rotational movement of a dial 152 into a linear movement by a pinion gear 153 and a rack gear 154, so that coupling plates 155 and 255 provided in the operating unit 100 and the insertion unit 200 are coupled to each other. In this case, the coupling plates 155 and 255 include a first coupling plate 155 provided in the operating unit 100 and a second coupling plate 255 provided in the insertion unit 200.

Meanwhile, in the present invention, connection of wires 130 and 230 may be achieved by wire frames 156 and 256. That is, the wires 130 and 230 include an operating wire 130 provided in the operating unit 100 and a bending wire 230 provided in the insertion unit 200, and these are respectively coupled to a first wire frame 156 provided in the operating unit 100 and a second wire frame 256 provided in the insertion unit 200. At this time, the first wire frame 156 and the second wire frame 256 are detachably coupled to each other, and in a coupled state, they may linearly reciprocate within the coupling plates 155 and 255.

This will be described in detail later.

The operating unit 100 is provided so that an operator can operate it. The operating unit 100 may be provided so that an operator can grip it and operate it. The operating unit 100 may operate to bend the insertion unit 200 through an operator's operation.

The insertion unit 200 is inserted into a human body by operation of the operating unit 100 and is provided to image the inside of the human body. The insertion unit 200 may be inserted into the human body, may be provided to be bendable according to operation of the operating unit 100, may be moved along an internal structure of the human body, and may illuminate and image the inside of the human body.

Although not shown, the endoscope 1 according to an embodiment of the present invention may further include a connector adapter and a universal cord.

Specifically, the endoscope 1 includes a connector adapter for connection to an external device, and the connector adapter may be connected to the operating unit 100 by a universal cord. The external device connected to the connector adapter may be an image processing device. Through this, an image imaged by the insertion unit 200 may be displayed on the external image processing device. Through this, the operator can operate the operating unit 100 while watching the image imaged by the insertion unit 200 on the image processing device.

The operating unit 100 includes an operating unit housing 110, an operating knob 120, an operating wire 130, and a first coupling part 150.

The operating unit housing 110 provides a structure for a practitioner to grip it, and may accommodate an operating wire 130 and the like for driving the insertion unit 200 inside.

Specifically, an operating knob 120 is disposed at an upper portion of the operating unit housing 110, a first coupling part 150 is disposed at one side in a longitudinal direction of the operating knob 120, and a sprocket and a chain linked to rotation of the operating knob 120 and an operating wire 130 are accommodated inside the operating unit housing 110, and a channel and the like may be further accommodated therein.

The operating knob 120 may control the insertion unit 200 while rotating by an operator's operation. The operating knob 120 may include two or more knobs. For example, the operating knob 120 may be provided in a form where a first knob 121 and a second knob 122 are stacked along a vertical direction. The first knob 121 and the second knob 122 are respectively connected to a first sprocket and a second sprocket (which will be described later), and configured to be able to rotate independently of each other. The first knob 121 and the second knob 122 have a shape in which a plurality of teeth protrude so that a practitioner can easily grip and rotate them.

Meanwhile, the first knob 121 may rotate independently while the second knob 122 maintains a stopped state. Similarly, the second knob 122 may rotate independently while the first knob 121 maintains a stopped state. The first knob 121 controls vertical movement of the illumination and imaging unit 210. That is, when the first knob 121 is rotated in a clockwise or counterclockwise direction, a distal end of the illumination and imaging unit 210 is bent upward or downward accordingly. The second knob 122 controls horizontal movement of the illumination and imaging unit 210. That is, when the second knob 122 is rotated in a clockwise or counterclockwise direction, the distal end of the illumination and imaging unit 210 is bent to the left or right accordingly.

By operating the first knob 121 and the second knob 122 in this way, the rotation direction of the illumination and imaging unit 210 can be arbitrarily adjusted. According to an embodiment, there may be a need to fix the illumination and imaging unit 210 in a state of being bent at a specific angle, and for this purpose, a fixing knob 123 is provided above the second knob 122. When the fixing knob 123 is rotated, the first sprocket and the second sprocket are fixed so as not to rotate, and accordingly, the distal end of the illumination and imaging unit 210 also maintains a fixed state.

Although not shown, two sprockets are rotatably mounted below the first knob 121 and the second knob 122 in linkage with rotation of the first knob 121 and the second knob 122. And a chain is provided which moves in engagement with the respective sprockets. The chains are responsible for vertical movement and horizontal movement of the illumination and imaging unit 210, respectively, and they can move independently of each other.

Both ends of the chain are coupled to the operating wire 130, respectively. Movement of the operating wire 130 is guided through a guide structure formed inside the operating unit housing 110.

The operating wire 130 is coupled to the first coupling part 150. Specifically, the operating wire 130 is coupled by the first wire frame 156 of the first coupling part 150. The first wire frame 156 is slidably mounted within a limited range of the first coupling plate 155.

Meanwhile, the first wire frame 156 is coupled to the second wire frame 256 of the insertion unit 200, whereby the operating wire 130 can be connected to the bending wire 230.

Accordingly, the operating wire 130 can perform a bending operation of the insertion tube 220 according to the operation of the operating knob 120.

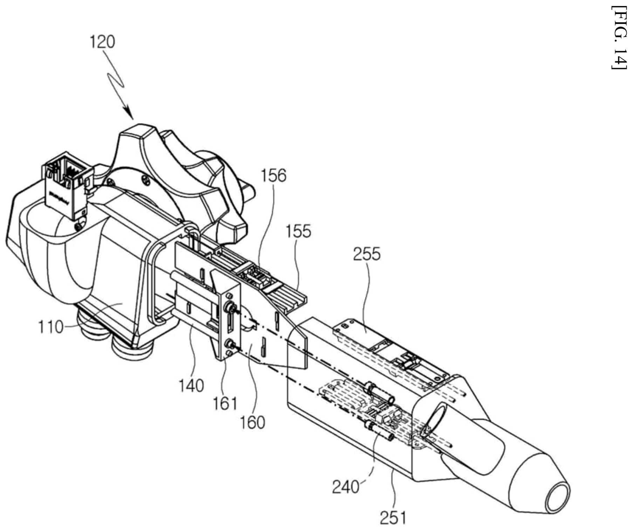

Meanwhile, FIG. 14 is a perspective view of FIG. 2 as viewed from another angle.

Referring to FIG. 14, the operating unit 100 may be provided with a channel 140 for providing air and water to an affected area during a procedure. For example, the channel 140 may be a supply channel and a suction channel. In this case, the supply channel may be an air supply channel and/or a water supply channel. The air supply channel, the water supply channel, and the suction channel have a substantially cylindrical shape and have an empty interior, so that when coupled with the illumination and imaging unit 210, they are inserted into the illumination and imaging unit 210 to ensure that water, air, or the like can be stably supplied to the procedure or examination site without leakage.

Meanwhile, although not shown, the operating unit 100 may further include a connection terminal. The connection terminal may be provided to be electrically connected to the illumination and imaging unit 210 so that data obtained by a camera or the like provided at a distal end of the illumination and imaging unit 210 can be transmitted to an external device. For example, the connection terminal may be an HDMI terminal for transmitting audio and video.

The channel 140 and the connection terminal may be disposed below the wires 130 and 230. For example, the channel 140 and the connection terminal are accommodated in a space formed of the operating unit housing 110, the first coupling part body 151, and the second coupling part body 251, but may be disposed further from the operating knob 120 than the operating wire 130.

At this time, a region where the operating wire 130 and the bending wire 230 move and a region where the channel 140 and the connection terminal are disposed may be divided by a support plate 160. The support plate 160 may partition an internal space of the operating unit 100 into upper and lower parts, and may include a channel support part 161 coupled with the channel 140 to support the channel 140, thereby guiding a fixed-position connection of the channel 140 when the operating unit 100 and the insertion unit 200 are coupled. That is, when the first coupling part 150 and the second coupling part 250 are coupled, the channel 140 of the operating unit 100 may be connected to the channel 240 of the insertion unit 200.

The channel support part 161 is formed to extend downward from a lower surface of the support plate 160, and a circular hole may be formed such that the channel 140 passes therethrough to be supported. In this case, the number of the holes may be formed to correspond to the number of the channels 140.

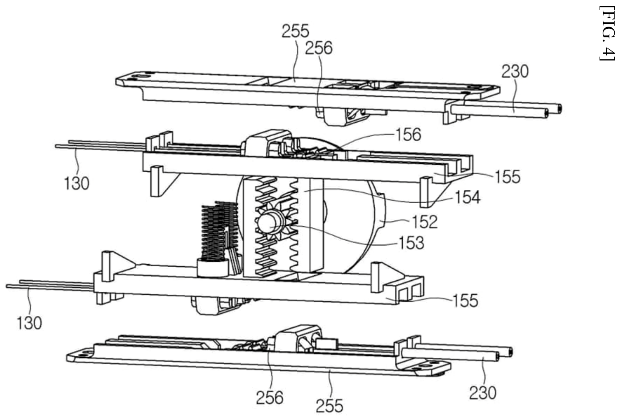

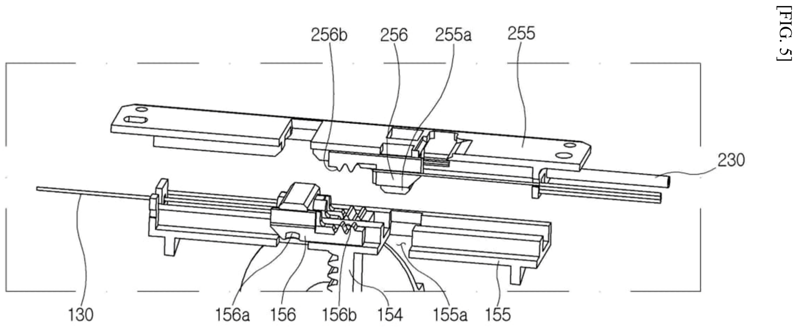

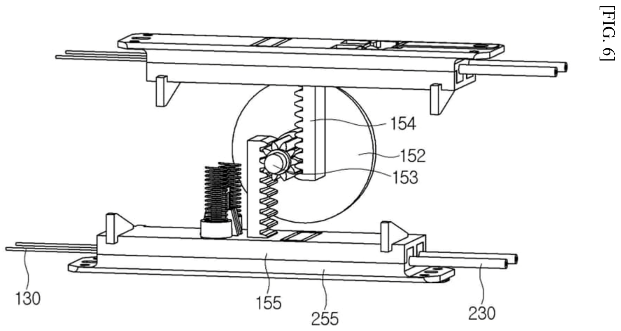

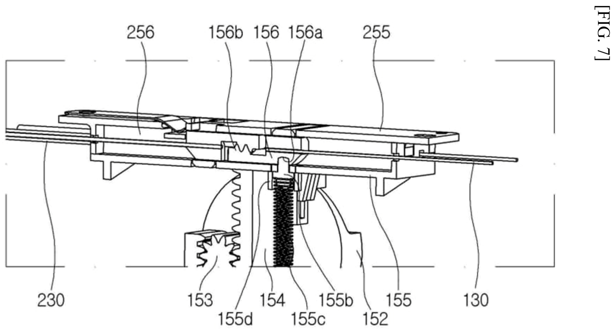

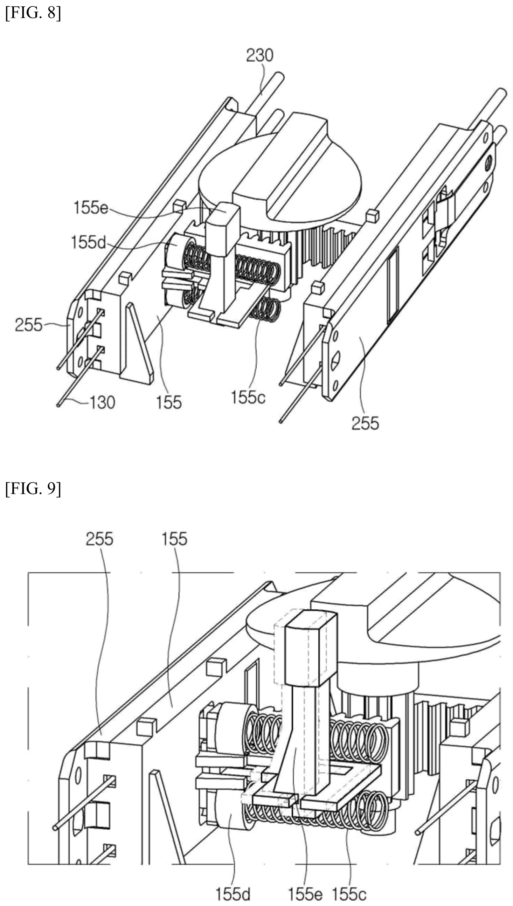

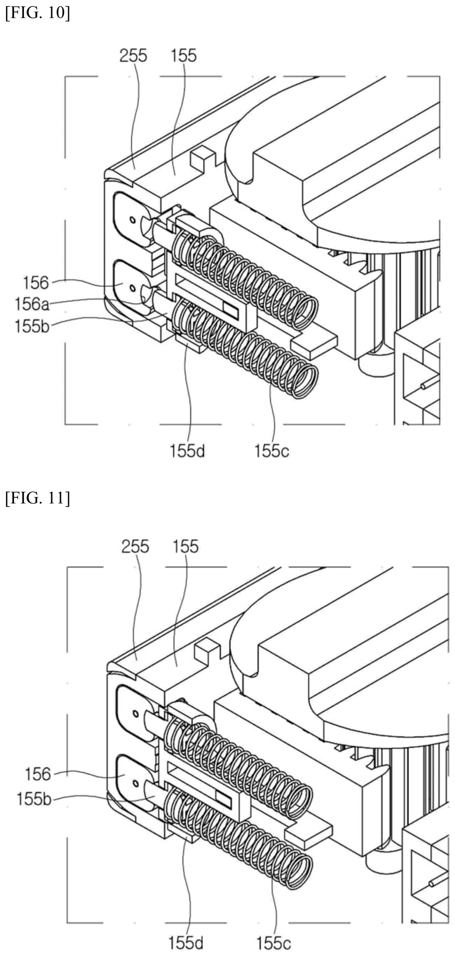

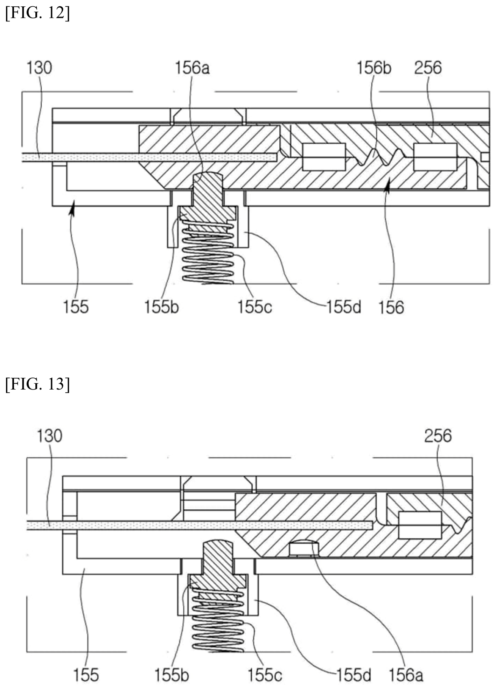

Meanwhile, FIG. 4 is a view for explaining a coupling method of a first coupling part and a second coupling part in the endoscope according to an embodiment of the present invention, FIG. 5 is an enlarged view of FIG. 4 as viewed from another angle, FIG. 6 is a view for explaining a state in which the first coupling part and the second coupling part are coupled in the endoscope according to an embodiment of the present invention, and FIGS. 7 to 13 are views for explaining a structure for guiding an initial position of a wire frame in the endoscope according to an embodiment of the present invention.

Referring to FIGS. 4 to 13, the first coupling part 150 is described as follows.

The first coupling part 150 may be disposed at one end of the operating unit housing 110. The first coupling part 150 may be detachably coupled to the insertion unit 200 by an operation of a practitioner (user).

Specifically, the first coupling part 150 includes a first coupling part body 151, a dial 152, a pinion gear 153, a rack gear 154, and a first coupling plate 155.

The first coupling part body 151 is provided to engage and couple with the second coupling part 250 of the insertion unit 200. For example, the first coupling part body 151 may be a plate shape wrapping around three sides. As another example, the first coupling part body 151 may be a pair of plate shapes extending from the operating unit housing 110 in a direction of the insertion unit 200. Accordingly, the first coupling part body 151 may be coupled with the second coupling part body 251 of the second coupling part 250.

As a result, the first coupling part body 151 and the second coupling part body 251 may be fitted into each other.

The dial 152 may be provided so that a user can grip and rotate it. The dial 152 may be rotatably coupled to the operating unit housing 110.

Specifically, the dial 152 may be exposed to the outside of the operating unit housing 110. For example, the dial 152 may include a disc-shaped rotating plate and a gripping part which protrudes in a rib shape from the rotating plate so that a user can hold and rotate it.

The pinion gear 153 is disposed inside the first coupling part body 151, and may be rotated in linkage with the rotation of the dial 152.

Meanwhile, the pinion gear 153 may be connected to the dial 152. The pinion gear 153 may be connected to the dial 152 by passing through the first coupling part body 151. For example, the pinion gear 153 and the dial 152 may be formed integrally.

Gear teeth are formed on an outer circumferential surface of the pinion gear 153, and may be engaged with a rack gear 154 (which will be described later). At this time, the pinion gear 153 may be engaged with a pair of rack gears 154. The pair of rack gears 154 may be disposed at positions facing each other with respect to the pinion gear 153.

The rack gear 154 is engaged with the pinion gear 153, and may be linearly moved according to the rotation of the pinion gear 153. At this time, the pair of rack gears 154 facing each other may be linearly moved in opposite directions according to the rotation of the pinion gear 153.

The pair of rack gears 154 may be disposed between the pair of first coupling part bodies 151. In this case, the pair of rack gears 154 may be disposed along a direction intersecting a direction in which the first coupling part body 151 is formed, and may be linearly reciprocated along a direction intersecting the direction in which the first coupling part body 151 is formed.

The pair of rack gears 154 may be respectively coupled to the first coupling plate 155. The first coupling plate 155 may be formed along a direction intersecting a longitudinal direction of the rack gear 154.

The first coupling plate 155 may be disposed between a pair of surfaces facing each other among the first coupling part bodies 151. And, the first coupling plate 155 may be disposed between the pair of second coupling part bodies 251. Meanwhile, the first coupling plate 155 may be disposed inside the second coupling part body 251, and disposed at a position facing the second coupling part body 251.

In addition, the first coupling plate 155 may be disposed at a position facing the second coupling plate 255. The first coupling plate 155 is moved together with the rack gear 154 according to the movement of the rack gear 154, and may be coupled with the second coupling plate 255.

For example, the first coupling plate 155 may be formed in a square plate shape having a predetermined thickness. In this case, a fixing part 155a for fixing the coupling with the second coupling plate 255 may be formed on the first coupling plate 155. For example, the fixing part 155a may be formed in a hole shape and coupled with a protrusion-shaped fixing part 255a formed on the second coupling plate 255.

Meanwhile, the first wire frame 156 may be disposed on the first coupling plate 155. The first wire frame 156 may be coupled to the first coupling plate 155 so as to be linearly reciprocable. For example, a guide groove for guiding the linear movement of the first wire frame 156 may be formed in the first coupling plate 155, and the first wire frame 156 may be disposed in the guide groove.

Meanwhile, the operating wire 130 is coupled to the first wire frame 156. For example, the operating wire 130 is coupled to one side in a longitudinal direction of the first wire frame 156, and may be provided to be linearly reciprocable along the longitudinal direction.

Meanwhile, the first wire frame 156 disposed on the first coupling plate 155 may be coupled to the second wire frame 256 disposed on the second coupling plate 255. For example, a frame coupling part 156b may be formed at the other side in the longitudinal direction of the first wire frame 156. The frame coupling part 156b may have a shape in which a plurality of ribs and grooves are alternately formed. The frame coupling part 156b may be formed in a shape corresponding to a frame coupling part 256b of the second wire frame 256. That is, the first wire frame 156 and the second wire frame 256 may be coupled in an engaged state with each other.

The first wire frame 156 may be disposed between the first coupling plate 155 and the second coupling plate 255 in a state coupled with the second wire frame 256. Therefore, the first wire frame 156 and the second wire frame 256, in the coupled state, can linearly reciprocate along the longitudinal direction of the endoscope 1.

Through this, the operating wire 130 coupled to the first wire frame 156 and the bending wire 230 coupled to the second wire frame 256 can move in linkage with each other, and the insertion tube 220 can be bent according to the operation of the operating knob 120.

Meanwhile, the first coupling plate 155 may further include a locking pin 155b, a spring 155c, and a locking holder 155d.

The locking pin 155b is disposed on the first coupling plate 155, and may be accommodated in a fixing groove 156a formed in the first wire frame 156 according to reciprocating movement. The locking pin 155b may include a head part accommodated in a locking holder 155d (which will be described later) and a pin part accommodated in the fixing groove 156a by passing through the locking holder 155d. At this time, the head part may be formed in a shape having a diameter larger than that of the pin part. Accordingly, a range of movement of the locking pin 155b may be limited by the locking holder 155d.

Meanwhile, the locking pin 155b may be biased toward the first wire frame 156 through the spring 155c. Specifically, the head part of the locking pin 155b may contact one end of the spring 155c. Accordingly, the locking pin 155b may be elastically supported by the spring 155c.

The other end of the spring 155c may be fixed to the first coupling part body 151.

The locking holder 155d is detachably coupled to the first coupling plate 155, and may accommodate the locking pin 155b so as to be reciprocable.

The locking holder 155d is coupled with a locking lever 155e, and may be reciprocated by an operation of the locking lever 155e. At this time, the locking holder 155d may be detachably coupled to the first coupling plate 155 according to the operation of the locking lever 155e.

The locking holder 155d may be formed to accommodate the locking pin 155b therein. For example, the locking holder 155d is formed in a cylindrical shape, wherein one side in a longitudinal direction may be formed corresponding to a size of the head part of the locking pin 155b, and the other side in the longitudinal direction may be formed corresponding to a size of the pin part of the locking pin 155b. That is, one side in the longitudinal direction of the locking holder 155d may be formed to be larger than a diameter of the head part, and the other side in the longitudinal direction may be formed to be smaller than the diameter of the head part but larger than a diameter of the pin part.

Therefore, the pin part of the locking pin 155b may pass through the locking holder 155d and be accommodated in the fixing groove 156a of the first wire frame 156.

For example, a pair of locking holders 155d may be provided, and a lever coupling part coupled with the locking lever 155e may be provided between the pair of locking holders 155d. The lever coupling part may be formed in a long hole shape so that one side of the locking lever 155e can be coupled thereto.

Meanwhile, one side of the locking lever 155e is coupled to the locking holder 155d, and the other side may be exposed to the outside of the first coupling part body 151. Accordingly, a user can hold and operate the locking lever 155e.

Meanwhile, a fixing groove 156a may be formed in the first wire frame 156. The fixing groove 156a may be formed on a surface opposite to a surface on which the frame coupling part 156b is formed. That is, the frame coupling part 156b may be formed on a surface at a position facing the second wire frame 256, and the fixing groove 156a may be formed on the opposite surface thereof.

The fixing groove 156a may accommodate the locking pin 155b according to movement of the first wire frame 156. That is, when the first wire frame 156 is disposed at a preset initial position, the fixing groove 156a may be disposed to face the locking pin 155b. At this time, when a user operates the locking lever 155e, the locking pin 155b is moved along the locking holder 155d and may be inserted into the fixing groove 156a by passing through the first coupling plate 155.

Therefore, according to the present invention, after replacing the insertion unit 200, the user can rotate the operating knob 120 while the locking lever 155e is operated, and accordingly, a position where the locking pin 155b and the fixing groove 156a are coupled can be found as the first wire frame 156 linearly moves (slidably moves).

As a result, in the present invention, there is an effect that an initial position of the operating wire 130 can be found and maintained after the insertion unit 200 is replaced.

The insertion unit 200 is detachably coupled to the operating unit 100, and at least a portion thereof is inserted into a human body to image the inside of the human body.

The insertion unit 200 includes an illumination and imaging unit 210, an insertion tube 220, a bending wire 230, and a second coupling part 250. At this time, the illumination and imaging unit 210 is disposed at one side in a longitudinal direction of the insertion unit 200, and the second coupling part 250 is disposed at the other side thereof. In addition, the illumination and imaging unit 210 and the second coupling part 250 may be connected by the insertion tube 220, and at least a portion of the bending wire 230 may pass from the second coupling part 250 to the insertion tube 220.

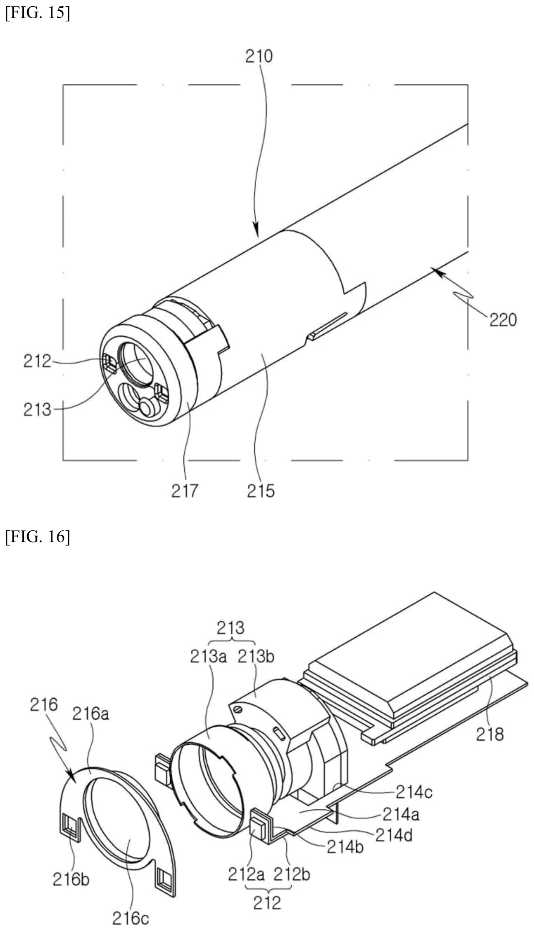

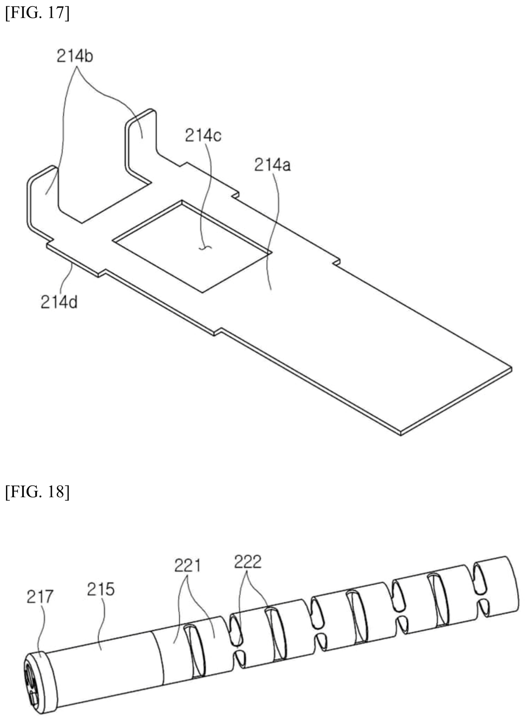

Meanwhile, FIG. 15 is a perspective view for explaining an illumination and imaging unit in the endoscope according to an embodiment of the present invention, FIG. 16 is a perspective view for explaining an internal structure of the illumination and imaging unit in the endoscope according to an embodiment of the present invention, and FIG. 17 is a view for explaining a heat sink in the endoscope according to an embodiment of the present invention.

The illumination and imaging unit 210 is described as follows with reference to FIGS. 15 to 17.

The illumination and imaging unit 210 is inserted into a human body to illuminate the inside of the human body and image the inside of the human body.

The illumination and imaging unit 210 includes an illumination and imaging unit body 211, an illumination module 212, and an imaging module 213.

The illumination and imaging unit body 211 forms an exterior of the illumination and imaging unit 210 and is configured to be inserted into the human body. For example, the illumination and imaging unit body 211 may have an illumination module 212 and an imaging module 213 disposed at one side in a longitudinal direction, and the other side in the longitudinal direction may be open to be connected to the insertion tube 220.

The illumination module 212 may be coupled to an end cap 217. The illumination module 212 can illuminate light. The illumination module 212 may include an illumination lens 212a capable of illuminating light upon receiving power. For example, the illumination lens 212a may be an LED (Light Emitting Diode).

Meanwhile, in the present embodiment, a plurality of illumination lenses 212a may be provided. Specifically, in the present embodiment, an even number of illumination lenses 212a may be provided. For example, a pair of illumination lenses 212a may be disposed line-symmetrically. This has an effect of preventing illuminance on an object from being asymmetrical at a short distance and preventing a gap in illumination from occurring.

At this time, in the present embodiment, the pair of illumination lenses 212a may be symmetrically disposed with respect to the imaging module 213. That is, in the present embodiment, the pair of illumination lenses 212a may be symmetrically disposed, and the imaging module 213 may be disposed on an axis of symmetry.

In this case, the pair of illumination lenses 212a may have different Correlated Color Temperatures (CCT). Through this, a target wavelength distribution of light can be created.

In addition, the illumination module 212 may further include an illumination support part 212b supporting the pair of illumination lenses 212a. The illumination support part 212b is formed in a flat plate shape, wherein a pair of distal ends are symmetrically extended and bent upward. The pair of illumination lenses 212a may be coupled to the pair of bent extended parts.

For example, the illumination support part 212b may be a flexible printed circuit board (FPCB). An image sensor may be mounted on the FPCB. Since a plurality of illumination lenses 212a are utilized as an illumination part, not only is it easy to adjust illuminance, but also an amount of irradiated light can be significantly increased compared to conventional halogen lamps or the like, thereby further improving clarity of an image obtained through the image sensor.

Meanwhile, the imaging module 213 can image the inside of a human body. The imaging module 213 may image an image of the inside of the human body upon receiving power and transmit captured image data. For example, the imaging module 213 may include a camera 213a and an image processor 213b. In this case, the camera 213a may be disposed at one end (distal end) in the longitudinal direction of the illumination and imaging unit 210, and the image processor 213b may be coupled with the camera 213a to process an image received from the camera.

Through this, the endoscope 1 of the present invention can illuminate light through the illumination module 212 while being inserted into the human body and image the inside of the human body through the imaging module 213.

The heat sink 214 may be disposed inside the illumination and imaging unit body 211. The heat sink 214 may be coupled with the illumination module 212 and/or the imaging module 213.

The heat sink 214 includes a heat sink body 214a, an illumination contact part 214b, an imaging module receiving hole 214c, and a heat transfer part 214d.

The heat sink body 214a may be provided to transfer heat. The heat sink body 214a may be formed of a material capable of transferring heat. For example, the heat sink body 214a may be formed of a metal material.

The heat sink body 214a is formed in a flat plate shape, wherein one end in a longitudinal direction (long-axis direction) is connected to the illumination contact part 214b, heat transfer parts 214d are connected to both sides in a width direction (short-axis direction), and an imaging module receiving hole 214c may be formed therein.

The illumination contact part 214b is formed to extend from one end in the longitudinal direction of the heat sink body 214a and may contact the illumination module 212. The illumination contact part 214b may be formed corresponding to a shape of the illumination support part 212b. For example, the illumination contact part 214b may be formed to extend from a distal end in the longitudinal direction of the heat sink body 214a and then bent and extend upward. Accordingly, the illumination contact part 214b may contact the illumination support part 212b. Through this, heat generated from the illumination module 212 can be transferred to the heat sink 214 via the illumination contact part 214b.

The imaging module receiving hole 214c is formed in the heat sink body and may accommodate the imaging module 213 so as to pass therethrough. For example, the imaging module receiving hole 214c may be formed in a rectangular hole shape, where a portion of the image processor 213b passes therethrough and another portion of the image processor 213b may be in contact. Through this, heat generated from the imaging module 213 can be transferred to the heat sink 214.

Meanwhile, a printed circuit board 218 may contact the other end in the longitudinal direction of the heat sink 214. The printed circuit board 218 may control the illumination module 212 and/or the imaging module 213. A circuit capable of controlling the illumination module 212 and/or the imaging module 213 may be mounted on the printed circuit board 218. Through this, heat generated from the circuit can be transferred to the heat sink 214.

The heat transfer part 214d may be formed to extend from both ends in a width direction (short-axis direction) of the heat sink body 214a. At this time, a width of the heat sink body 214a is smaller than a diameter of the heat dissipation chassis 215, and a combined width of the heat sink body 214a and the heat transfer part 214d may be equal to or slightly larger than the diameter of the heat dissipation chassis 215. Therefore, the heat transfer part 214d may contact the heat dissipation chassis 215. Accordingly, heat of the heat transfer part 214d can be transferred to the heat dissipation chassis 215.

The heat dissipation chassis 215 is provided to wrap around an outer circumferential surface of the illumination and imaging unit body 211 and contacts the heat sink 214 to dissipate heat of the illumination module 212 and/or the imaging module 213.

For example, the heat dissipation chassis 215 may be a coil pipe made of a spring material and can release heat generated from the illumination module 212 and/or the imaging module 213.

Meanwhile, in another embodiment of the present invention, the heat dissipation chassis 215 and the illumination and imaging unit body 211 may be formed integrally.

Meanwhile, the illumination and imaging unit 210 may further include a heat shield plate 216 that is disposed at one side in the longitudinal direction of the illumination and imaging unit 210 and blocks heat generated from the illumination module 212.

The heat shield plate 216 is coupled to the end cap 217, and may be disposed between the end cap 217 and the illumination module 212 along a longitudinal direction of the illumination and imaging unit 210.

The heat shield plate 216 may include a shield plate body 216a formed in a semi-circular shape, an illumination cover part 216b symmetrically disposed on the shield plate body 216a and disposed to face the illumination module 212, and a camera cover part 216c disposed on an axis of symmetry of the pair of illumination cover parts 216b and disposed at a position facing the imaging module 213.

The heat shield plate 216 may prevent heat generated from the illumination module 212 from being transferred to a human body. In this case, the heat shield plate 216 may be formed of a material through which light can pass.

An end cap 217 is provided at a longitudinal end (distal end) of the illumination and imaging unit 210. The end cap 217 has a cylindrical structure and is coupled to an end of the insertion unit 200. In this case, the illumination module 212 and the imaging module 213 may be coupled to the end cap 217. In addition, the heat shield plate 216 may be coupled to the end cap 217.

The end cap 217 may be provided with light transmission windows at a plurality of points. The light transmission window is made of a plate material of glass or a transparent material, and has a structure capable of transferring light irradiated from the illumination module 212 to the outside.

Meanwhile, the end cap 217 may be formed with a plurality of channel receiving tubes into which the channel 240 is inserted. The inside of each channel receiving tube has a hollow cylindrical shape and is formed to have an inner diameter corresponding to an outer diameter of each insertion channel 140. And, an O-ring for preventing leakage of supplied water or air may be provided inside.

Meanwhile, a connection terminal may be provided in the illumination and imaging unit 210. The connection terminal may be provided corresponding to the connection terminal of the operating unit 100. That is, a male terminal may be provided in the operating unit 100, and a female terminal may be provided in the insertion unit 200.

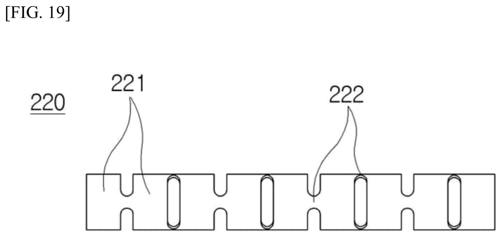

FIGS. 18 and 19 are views for explaining a bending part in an endoscope according to an embodiment of the present invention.

Referring to FIGS. 18 and 19, a structure of a bending part of the endoscope according to an embodiment of the present invention is described as follows.

The insertion tube 220 connects the illumination and imaging unit 210 and the second coupling part 250, and may be bent according to an operation of the operating unit 100.

Specifically, the insertion tube 220 may be bent according to movement of the bending wire 230.

Meanwhile, a plurality of channels 240 and terminals may be accommodated inside the insertion tube 220.

The insertion tube 220 may include bending parts 221 and 222 and an insertion tube portion 224.

The bending parts 221 and 222 may be configured to be bent according to movement of the bending wire 230. The bending parts 221 and 222 may be configured to be bent by reciprocating movement of the bending wire 230 moving according to an operation of the operating unit 100.

The bending parts 221 and 222 include a plurality of bending sections 221 rotated relative to each other and connection portions 222 connecting the plurality of bending sections 221.

In this case, the bending section 221 and the connection portion 222 may be formed of different materials. Specifically, the bending section 221 may be molded of a first material, and the connection portion 222 may be molded of a second material different from the first material.

For example, the first material may be a polycarbonate (PC)-based material. Polycarbonate (PC) has an advantage of excellent mechanical strength.

Further, the second material may be a polyurethane (PU)-based material. For example, the second material may be thermoplastic polyurethane (TPU). Polyurethane (PU) has advantages of high durability and flexibility.

That is, in the present invention, the bending section 221 may be molded of a material having high rigidity, and the connection portion 222 may be molded of a material having high flexibility.

Meanwhile, the bending section 221 may be formed in a ring shape. Specifically, the bending section 221 may be formed in a ring shape having a predetermined thickness along a radial direction and having a predetermined length along an axial direction. In this case, the axial length may be larger than the radial thickness of the bending section 221. Accordingly, the bending section 221 may also be referred to as a cylindrical shape having a predetermined length.

A plurality of the bending sections 221 are arranged along the axial direction, and the plurality of the bending sections 221 may be connected to each other by the connection portion 222. That is, in a state where no external force is applied, the plurality of bending sections 221 may be arranged in a line on the same axis, and internal spaces of the plurality of bending sections 221 may be arranged to communicate with each other. Through this, a plurality of bending wires 230, channels 240, and terminals may be disposed to pass through the inside of the plurality of bending sections 221.

Meanwhile, the connection portions 222 may be formed as a pair at both longitudinal ends of the bending section 221, respectively.

Four connection portions 222 are formed in one bending section 221, wherein a pair of connection portions 222 may be formed at one longitudinal end of each bending section 221, and another pair of connection portions 222 may be formed at the other longitudinal end thereof.

In this case, the connection portions 222 may be disposed with a constant phase difference along a circumferential direction of the bending section 221. For example, the pair formed at one end and the pair formed at the other end of the bending section 221 may be alternately disposed with a phase difference of 90 degrees from each other along the circumferential direction. That is, a pair of connection portions 222 formed at an end in the same direction are disposed with a phase difference of 180 degrees along the circumferential direction, but may be disposed with a phase difference of 90 degrees with respect to a pair formed at an end in the other direction.

The connection portions 222 may serve as an axis of rotation for the bending section 221. In the present invention, the rigidity of the bending section 221 formed of the first material is larger than the rigidity of the connection portions 222 formed of the second material. In addition, a radial thickness and an axial length of the bending section 221 are larger than those of the connection portions 222. Therefore, when an external force is applied to the plurality of bending sections 221 due to movement of the bending wire 230 or the like, deformation may not occur in the bending section 221 having relatively high rigidity, but elastic deformation may occur in the connection portions 222 having relatively low rigidity and high flexibility. Accordingly, when an external force is applied to the bending parts 221 and 222, the bending section 221 may be rotated relative to each other as the connection portions 222 are bent.

Therefore, the bending part 221, 222 bending operation may be implemented as the plurality of bending sections 221 are rotated relative to each other with the connection portions 222 serving as an axis.

Therefore, according to the present invention, it is possible to manufacture an insertion tube 220 that is capable of a bending operation at a low cost by providing the bending parts 221 and 222 consisting of the polycarbonate-based bending sections 221 having rigidity and the urethane-based connection portions 222 having flexibility.

Meanwhile, a bending part of a conventional endoscope was manufactured by producing a plurality of articulated structures made of a metal material and connecting them using welding or rivets.

However, since the articulated structure formed of metal as described above incurs high manufacturing costs, there is a limitation in that it is not suitable for use in a disposable endoscope that is replaced after a single use.

To solve this, in the endoscope 1 of the present invention, the bending section 221 and the connection portion 222 may be integrally formed. In this case, the bending section 221 and the connection portion 222 may be formed by being simultaneously injected. That is, in the present invention, the bending parts 221 and 222 may be manufactured by simultaneously injecting the bending section 221 made of the first material and the connection portion 222 made of the second material using a single mold.

Through this, since the bending part may be produced in a single process using a relatively low-cost material, economic efficiency suitable for application to a disposable endoscope may be provided.

Meanwhile, an insertion tube portion 224 may be disposed at the other longitudinal side of the insertion tube 220. The insertion tube portion 224 is formed in a bendable tube shape, and the bending wire 230, the channel 240, and a terminal may pass therethrough. Although not shown, a coil pipe may be provided in the insertion tube portion 224 to provide an elastic force to the insertion tube portion 224.

Meanwhile, the bending wire 230 may receive an operating force of the operating knob 120 through the second coupling part 250, and may perform a bending operation of the insertion tube 220 through linear reciprocating movement.

One longitudinal end of the bending wire 230 is coupled to the illumination and imaging unit 210, and the other longitudinal end thereof may be coupled to the second wire frame 256. At this time, the bending wire 230 may pass through internal spaces of the plurality of bending sections 221.

Accordingly, on a side where the bending wire 230 is pulled, the bending sections 221 may be rotated relative to each other such that a gap between the plurality of bending sections 221 is narrowed. On the other hand, on a side where the bending wire 230 is loosened (lengthened), the bending sections 221 may be rotated relative to each other such that the gap between the plurality of bending sections 221 is widened. Accordingly, the plurality of bending sections 221 may form a bent shape.

Meanwhile, referring to FIGS. 4 to 13, the second coupling part 250 is detachably coupled to the first coupling part 150 of the operating unit 100. The second coupling part 250 includes a second coupling part body 251, a second coupling plate 255, and a second wire frame 256.

One longitudinal side of the second coupling part body 251 is coupled to the insertion tube 220, and the other longitudinal side thereof may be coupled to the first coupling part 150. In this case, the one longitudinal side of the second coupling part body 251 may be coupled to the other longitudinal side of the insertion tube 220, and the other longitudinal side of the second coupling part body 251 may be provided to be detachably couplable to the first coupling part body 151.

The second coupling part body 251 may be formed in a shape in which a diameter narrows toward one longitudinal end (distal end). As a result, the one longitudinal end of the second coupling part body 251 may be formed in a shape couplable to the insertion tube portion 224 of the insertion tube 220 to be connected thereto.

The other longitudinal side of the second coupling part body 251 may be provided to engage and couple with the first coupling part 150. For example, the other longitudinal side of the second coupling part body 251 may be in a plate form having three surfaces extending in a direction toward the operating unit 100. As another example, the other longitudinal side of the second coupling part body 251 may be in a form of a pair of plates extending in the direction toward the operating unit 100. Accordingly, the second coupling part body 251 may be coupled to the first coupling part body 151 of the first coupling part 150.

As a result, the second coupling part body 251 may be fitted into the first coupling part body 151. That is, the second coupling part body 251 may be coupled to the first coupling part body 151 to accommodate the first coupling plate 155, the first wire frame 156, the second coupling plate 255, and the second wire frame 256 therein.

The second coupling plate 255 may be disposed between a pair of surfaces facing each other among the second coupling part body 251. The second coupling plate 255 is disposed inside the second coupling part body 251 and may be disposed at a position facing the second coupling part body 251.

In addition, the second coupling plate 255 may be disposed at a position facing the first coupling plate 155. In this case, when the first coupling plate 155 is moved according to movement of the rack gear 154, it may be coupled to the second coupling plate 255.

For example, the second coupling plate 255 may be formed in a square plate shape having a predetermined thickness. In this case, a fixing portion 255a for fixing the coupling with the first coupling plate 155 may be formed on the second coupling plate 255. For example, the fixing portion 255a may be formed in a protrusion shape to be coupled with a hole-shaped fixing portion 155a formed on the first coupling plate 155.

Meanwhile, the second wire frame 256 may be disposed on the second coupling plate 255. The second wire frame 256 may be coupled to the second coupling plate 255 so as to be linearly reciprocable. For example, a guide groove for guiding linear movement of the second wire frame 256 may be formed in the second coupling plate 255, and the second wire frame 256 may be disposed in the guide groove.

Meanwhile, the bending wire 230 is coupled to the second wire frame 256. For example, the bending wire 230 is coupled to one side in a longitudinal direction of the second wire frame 256, and may be provided to be linearly reciprocable along the longitudinal direction.

Meanwhile, the first wire frame 156 may be coupled to the second wire frame 256. For example, a frame coupling portion 256b may be formed at the other side in a longitudinal direction of the second wire frame 256. The frame coupling portion 256b may be in a form where a plurality of ribs and grooves are alternately formed. The frame coupling portion 256b may be formed in a shape corresponding to the frame coupling portion 156b of the first wire frame 156. That is, the first wire frame 156 and the second wire frame 256 may be coupled in an engaged state with each other.

An assembly in which the first wire frame 156 and the second wire frame 256 are coupled may be disposed inside where the first coupling plate 155 and the second coupling plate 255 are coupled. In this case, the first wire frame 156 is accommodated in a guide groove formed in the first coupling plate 155, and the second wire frame 256 may be accommodated in a guide groove formed in the second coupling plate 255. Therefore, when the first coupling plate 155 and the second coupling plate 255 are coupled, they may surround the assembly of the first wire frame 156 and the second wire frame 256. That is, the assembly of the first wire frame 156 and the second wire frame 256 may reciprocate along a longitudinal direction (front-rear direction), but four sides (up, down, left, right) thereof may be constrained by the first coupling plate 155 and the second coupling plate 255.

Through this, the operating wire 130 coupled to the first wire frame 156 and the bending wire 230 coupled to the second wire frame 256 may move in linkage with each other, and the insertion tube 220 may be bent according to an operation of the operating knob 120.

While the present invention has been described in detail through specific embodiments, these are for describing the present invention in detail, and the present invention is not limited thereto. It is apparent that modifications or improvements can be made by those of ordinary skill in the art within the technical spirit of the present invention.

All simple modifications or changes of the present invention fall within the scope of the present invention, and the specific scope of protection of the present invention will be made clear by the appended claims.

## Claims

1. An endoscope comprising:
an insertion unit including an illumination and imaging unit and an insertion tube;
and
an operating unit provided with an operating knob and configured to perform a bending operation of the insertion tube according to an operation of the operating knob,
wherein the insertion tube includes a bending part configured to be bent according to the operation of the operating unit,
**characterized in that** the bending part includes:
a bending section injection-molded of a first material; and
a connection portion injection-molded of a second material different from the first material and connecting a plurality of the bending sections.

2. The endoscope of claim 1, wherein the bending section is formed in a ring shape.

3. The endoscope of claim 1, wherein a pair of the connection portions are formed at both longitudinal ends of the bending section, respectively.

4. The endoscope of claim 3, wherein the connection portions are disposed with a constant phase difference along a circumferential direction of the bending section.

5. The endoscope of claim 1, wherein the plurality of the bending sections are rotated relative to each other about the connection portion as an axis.

6. The endoscope of claim 1, wherein the first material is polycarbonate.

7. The endoscope of claim 1, wherein the second material is thermoplastic polyurethane.

8. The endoscope of claim 1, wherein the bending section and the connection portion are integrally formed.

9. The endoscope of claim 8, wherein the bending section and the connection portion are formed by being simultaneously injected.

10. The endoscope of claim 1, wherein a wire configured to linearly reciprocate according to the operation of the operating knob passes through an inside of the bending section.

11. The endoscope of claim 1, wherein the first material has higher rigidity than the second material.

12. The endoscope of claim 1, wherein the second material has higher flexibility than the first material.
